# EUROPEAN PATENT APPLICATION

(11) **EP 3 679 952 A1**
(43) Date of publication of application: **15.07.2020**
(21) Application number: 19305035.8
(22) Date of filing: 10.01.2019
(51) Int. Cl.: A61K 47/54, A61K 47/55, A61K 47/69, A61P 35/00, C07D 309/14, C07D 405/04, C07D 305/14

(54) **NANOEMULSIONS ENCAPSULATING PRODRUGS**

(71) Applicant: Université de Strasbourg, 67000 Strasbourg (FR); CENTRE NATIONAL DE LA RECHERCHE SCIENTIFIQUE, 75016 Paris (FR); INSERM (Institut National de la Santé et de la Recherche Médicale), 75013 Paris (FR)
(72) Inventor: KLYMCHENKO, Andrii, 67400 ILLKIRCH (FR); NIKO, Yosuke, KOCHI, 780-8063 (JP); ANTON, Nicolas, 67115 PLOBSHEIM (FR); GOETZ, Jacky, 67700 HAEGEN (FR); VANDAMME, Thierry, 67400 ILLKIRCH-GRAFFENSTADEN (FR); BOUCHAALA, Redouane, 67400 ILLKIRCH-GRAFFENSTADEN (FR); ANDREIUK, Bohdan, New York NY 10028 (US); LEFEBVRE, Olivier, 67600 MUTTERSHOLTZ (FR)
(74) Representative: IPAZ

(57) **Abstract**

The present invention concerns nanoemulsions encapsulating a prodrug and bearing a targeting ligand, said nanoemulsion being formed by a natural oil and/or a synthetic oil and an amphiphilic surfactant, the methods for preparing these nanoemulsion, and the applications of these nanoemulsion.

## Description

The present invention concerns lipid nanoemulsions encapsulating a prodrug, their uses and their methods of preparation.

Lipid nanoemulsions constitute highly promising tool for drug delivery because they are (1) inexpensive and easy to produce; (2) composed of GRAS (generally recognized as safe) elements; and (3) biodegradable (McClements et al., Soft Matter, 2012; 8 (6): 1719-1729; Anton et al., Pharm Res. 2011; 28(5):978-985). However, a drug having medium lipophilicity, such as doxorubicin (DOX), has tendency to leak out rapidly from lipid nanoemulsion into biological media, so that the nano-emulsions cannot really reach the target tumor together with the encapsulated drug. Therefore, encapsulation of drugs without leakage and their release under pH control constitute key challenges in application of nano-emulsions for cancer therapy. The existing technologies of delivery of drugs, including doxorubicin, mainly use polymer nanoparticles (Kumari et al., Colloids Surf B Biointerfaces. 2010; 75(1):1-18) or liposomes (Barenholz et al., J Control Release. 2012; 160(2): 117-134; Allen et al., Adv Drug Deliv Rev. 2013; 65(1):36-48.).

Li et al. (J Vis Exp. 2016 Aug 2; (114): 10.3791/54338) disclosed a doxorubicin lipophilic pro-drug, doxorubicin-palmitic acid (DOX-PA). Said pro-drug is encapsulated in DSPE-PEG micelles. This method utilizes the self-assembly properties of the amphiphilic polymer (DSPE-PEG) to form core-shell structured micelles in an aqueous environment.

Câmara et al. (Nanomedicine 2017; vol.12, No. 15) disclosed an acid-sensitive lipidated, doxorubicin (Dox) prodrug (C16-Dox) to be entrapped in lipid nanoemulsion (NE-C16-Dox) as a nanocarrier to treat breast cancer. Said C16-Dox was formed by a 1-hexadecanethioether chain conjugated to aldoxorubicin. Said nanoemulsions were prepared using C16-Dox in castor oil as dispersed phase and PEG-35-castor oil as surfactant.

However, the experiences carried out with the nanoemulsions encapsulating prodrug of doxorubicin bearing a single alkyl chain, like those described in the prior art, show that this type of nanoemulsions is unstable and forms precipitation only a few days after the formulation.

By the way, loading efficiency of such single alkyl chain conjugated-doxorubicin in the nanoemulsions is still not satisfactory for therapeutic uses.

Thus, there is still necessity to develop new nanoemulsions with better drug-loading efficiency and better stability.

Recent works of the Inventors using fluorescent dyes as model drugs highlighted the importance of lipophilicity to prevent cargo leakage and ensure the stability of the nanoemulsion (Klymchenko et al., RSC Adv. 2012; 2(31), 11876-11886; Kilin et al., Biomaterials. 2014; 35(18):4950-7). In these studies, using combination of near-infrared dyes undergoing Forster Resonance Energy Transfer, it was shown that the lipid nanoemulsions can deliver their cargo directly into xenographted tumours with minimal loss of their cargo. These results show that nanoemulsions are promising drug carriers for therapeutic applications, but the drugs should be modified in order to ensure their highest possible lipophilicity.

Against all odds, the Inventors of the present application have developed a new approach to solve the problem of insufficient lipophilicity of currently used drugs by synthesizing a prodrug bearing at least two long alkyl chains or a sterol moiety.

It appears that said prodrug displays better loading efficiency in lipid nanoemulsion and is prone to remain inside lipid nanoemulsion without leakage in neutral pH condition. The release of the moiety having pharmaceutical or cosmetic activity can however be triggered at low pH, thanks to the presence of a biodegradable bond in said prodrug. Moreover, the nanoemulsion containing said prodrug displays a better stability over time compared to nanoemulsion containing a prodrug bearing a single alkyl chain. Thus, the present invention makes it possible to use lipid nanoemulsions as controlled vehicles for drug delivery into target tissues, such as tumors.

In another aspect of the invention, the Inventors also propose for the first time an *in situ* method "drug sponge" for formulating lipid nanoemulsion encapsulating a prodrug, wherein a lipid nanoemulsion encapsulating a lipophilic reactive compound acts as a sponge that accumulates the drug directly added to the medium and wherein the prodrug in reversible form is directly synthesized inside the nanoemulsion oily core. Thus this new method ensures efficient encapsulation of the drug and avoids organic synthesis of a prodrug and consecutive detoxification of the medium from reactive agents, eventual solvents and ketone-containing drugs.

Therefore, the first aspect of the present invention is to provide a nanoemulsion encapsulating a prodrug, said nanoemulsion comprising a natural oil and/or a synthetic oil and at least an amphiphilic surfactant, said prodrug being represented by formula I,

D-L-G Formula I

wherein:
- L is a biodegradable covalent bond chosen from a hydrazone, an imine, an ester, an amide, a carbamate, a carbonate, an acetal, a hemiaminal,
- D is a moiety having pharmaceutical, cosmetic or biological activity,
- G represents:
   (i) a derivative of sterol, whose polycyclic part is linked to the group L; or
   (ii) a group of formula A 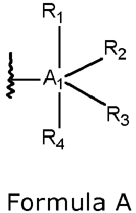 Wherein in A₁ is a spacer chosen from:
      ∘ a linear or branched alkyl chain comprising:
         ▪from 1 to 8 methylene bridge
         ▪ at least one 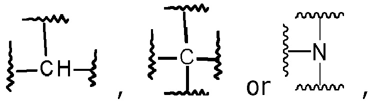
         ▪ optionally one or more linker groups chosen from -(C=O)-O-, -(C=O)-NH-, -O-(C=O)-NH-,-NH-, , -(C=O)-, -O-;
      ∘ a cyclo(C₃-C₇)alkyl eventually substituted by one or more linker groups chosen from -(C=O)-O-, -(C=O)-NH-, -O-(C=O)-NH-, -NH-, -(C=O)-, -O-; a monocyclic non-aromatic heterocyclic group eventually substituted by one or more linker groups chosen from -(C=O)-O-, -(C=O)-NH-, -O-(C=O)-NH-, -NH-, -(C=O)-, -O-; or a monocyclic aromatic group eventually substituted by one or more linker groups chosen from -(C=O)-O-, -(C=O)-NH-, -O-(C=O)-NH-, -NH-, -(C=O)-, -O-,
- R₁, R2, R3, and R4 are identical or different and each represent a (C4-C₂₀)alkyl or a hydrogen, wherein no more than two of R₁, R2, R3 and R4 are hydrogen on the same time.

Compared to the prodrug "C16-Dox" disclosed by Câmara et al. (Nanomedicine 2017; vol.12, No. 15), the prodrug of formula I of the present invention displays better loading efficiency in lipid nanoemulsion and better stability in nanoemulsion.

Without being bound to theory, it appears that the presence of moiety G in a prodrug of formula I significantly increases the lipophilicity of the prodrug and maintains it encapsulated inside the oily core of a nanoemulsion, and thus prevents the drug to leak from nanoemulsions in neutral pH conditions.

As used herein, the term "nanoemulsion" refers to oil-in-water dispersions comprising nanometer scale oil phase droplets in an aqueous phase.

In a particular embodiment, the diameter of the nanoemulsion is from 20 to 300 nm, preferably from 25 to 100 nm, more preferably from 30 to 80 nm.

Size distributions can be determined for example by the method of dynamic light scattering.

The terms "nanoemulsion" and "nanodroplet" are interchangeable in the present application.

The oily core in nanoemulsions can be formed by a natural oil, which is a vegetable oil or an animal oil, or be formed by a synthetic oil, or a mixture thereof.

Suitable animal oil are any natural lipid material extracted from animal and in liquid form at room temperature. Suitable animal oils can be fish oil.

Suitable vegetable oils are any natural lipid material that can be extracted from a plant and in liquid form at room temperature. Suitable vegetable oils include, but are not limited to Castor oil, Olive oil, Sunflower oil, Soybean oil, Colza oil, Peanut oil, Sesame oil, cotton oil.

In a preferred embodiment, the vegetable oil used in nanoemulsion formulation is rich in triglyceride derived from short-chain fatty acids or medium-chain fatty acids.

As used herein, the term "short-chain fatty acid" is referred to fatty acid with aliphatic tails of 2 to 6 carbon atoms.

As used herein, the term "medium-chain fatty acid" is referred to fatty acid with aliphatic tails of 7 to 12 carbons.

The term "synthetic oil" as used herein is referred to triglycerides artificially synthetized by a chemical method or fatty acid esters artificially synthetized by a chemical method.

Examples of synthetic oil include, but are not limited to medium chain triglycerides, polyoxylglycerides, glyceryl monocaprylates, and Vitamine E acetate. Particular examples of synthetic oil which can be cited are Labrafac® oil (medium chain triglyceride), Labrafil M1944CS (oleoyl polyoxyl-6 glycerides), Labrasol® (Caprylocaproyl polyoxyl-8 glycerides), Capmul MCM C8 (glyceryl monocaprylates.

The term "amphiphilic surfactant" as used herein is referred to a compound of bipolar structure having at least a lipophilic portion and at least a hydrophilic portion. In nanoemulsion, amphiphilic surfactant forms the interface membrane between oil phase droplet and aqueous phase.

Said surfactant can be an anionic surfactant (e.g. sodium dodecyl sulfate), a cationic surfactant (e.g. hexadecyltrimethylammonium bromide), a non-ionic surfactant, or a phospholipid (e.g. lecithins).

In a specific embodiment, said surfactant is a PEGlygated non-ionic surfactant, such as polyoxyethylene esters of 12-hydroxystearic acid, polyethylene glycol stearyl ether, polyoxyl hydrogenated castor oil, or polysorbates, castor oil PEG30 (Kolliphor® ELP), castor oil PEG40 (Kolliphor® RH40), hydroxystearate PEG15 (Kolliphor® HS15), Vitamine E TPGS, lecithins of soy, sunflower and egg yolk.

As used herein, the term "prodrug of formula (I)" is meant to a compound that can release in suitable condition, for example in low pH condition, a moiety having pharmaceutical, cosmetic or biological activity, by the degradation of biodegradable covalent bond L.

Examples of a moiety having biological activity can be but not limited to a molecule recognized by targeted cells, an antibody, an oligonucleotide, an agonist or antagonist of a natural biological molecule.

The term "biodegradable covalent bond" is meant to a covalent bond which can be broken down under certain physiological conditions in human body, tissues and cells. For example, hydrazone bond can be broken down in low pH condition that is often the case in tumoral cells.

In an embodiment of the present invention, said prodrug is a prodrug of a drug bearing a keto group, a hydroxyl group or an amino group.

In another embodiment of the present invention, said prodrug is a prodrug of an anticancer drug, in particular a prodrug of doxorubicin, paclitaxel, gemcitabine, rucaparib, docetaxel, decitabine, épirubicine, capecitabine, capecitabine, bendamustine, palbociclib, cytarabine, dabrafenib (GSK2118436), ribociclib, topotecan hcl, niraparib, ribociclib, lorlatinib, daunorubicin HCl, ibrutinib, mitomycin C, niraparib, methotrexate, amonafide, belizatinib, apitolisib (GDC-0980, RG7422), NU1025.

According to an embodiment, the nanoemulsions of the present invention encapsulate a prodrug of formula I:

D-L-G Formula I

Wherein G is a derivative of sterol.

The term "sterol" is meant to a group of lipids possessing a sterane ring with a hydroxyl group at the 3-position of the sterane ring.

Examples of derivatives of sterol which can be cited are cholesterol, phytosterols, ergosterol, or hydroxysteroid.

A particular example of prodrugs of formula I is given below. Said compound named Chol-Pax is a prodrug of paclitaxel.

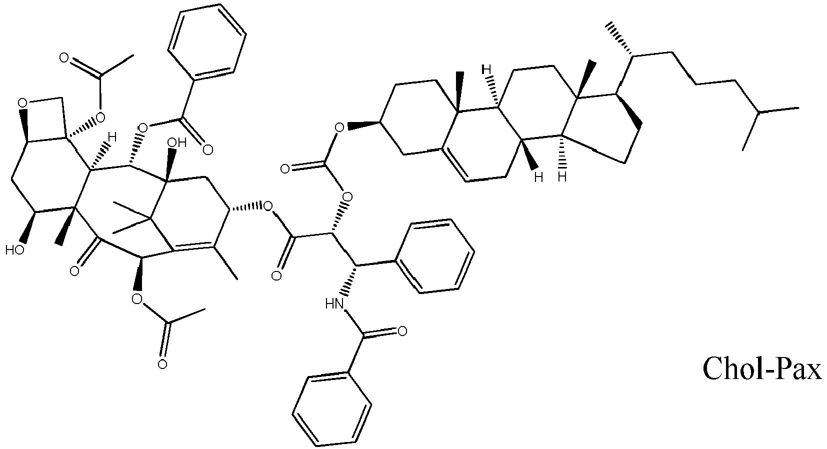

According to another embodiment, the nanoemulsions of the present invention encapsulate a prodrug of formula I'

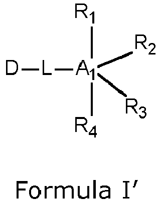

Wherein in A₁ is a spacer chosen from:
∘ a linear or branched alkyl chain comprising:
   ▪ from 1 to 8 methylene bridge
   ▪ at least one 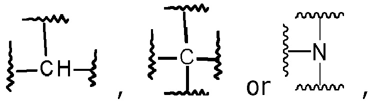
   ▪ optionally one or more linker groups chosen from -(C=O)-O-, -(C=O)-NH-, -O-(C=O)-NH-, -NH-, , -(C=O)-, -O-;
∘ a cyclo(C₃-C₇)alkyl eventually substituted by one or more linker groups chosen from -(C=O)-O-, -(C=O)-NH-, -O-(C=O)-NH-, -NH-, -(C=O)-, -O-; a monocyclic non-aromatic heterocyclic group eventually substituted by one or more linker groups chosen from -(C=O)-O-, -(C=O)-NH-, -O-(C=O)-NH-, -NH-, -(C=O)-, -O-; or a monocyclic aromatic group eventually substituted by one or more linker groups chosen from -(C=O)-O-, -(C=O)-NH-, -O-(C=O)-NH-, -NH-, -(C=O)-, -O-,

- L is a biodegradable covalent bond chosen from a hydrazone, an imine, an ester, an amide, a carbamate, a carbonate, an acetal, a hemiaminal
- D is a moiety having pharmaceutical, cosmetic or biological activity,
- R₁, R₂, R₃, and R₄ are identical or different and each represent a (C4-C₂₀)alkyl or a hydrogen, wherein no more than two of R₁, R₂, R₃ and R₄ are hydrogen on the same time.

The term "methylene bridge", as used herein, is referred to group -CH₂-.

The term "cyclo(C₃-C₇)alkyl", as used herein, is meant to a cyclic saturated carbon-based ring composed of from 3 to 7 carbon atoms. Examples of these cycloalkyl include cyclopropyl, cyclobutyl, cyclopentyl, cyclohexyl, cycloheptyl.

The term "monocyclic non-aromatic heterocyclic group" as used herein, is meant to a monocyclic saturated group which ring is formed by 3 to 10 atoms, wherein at least one atom besides carbon atoms is chosen from N, S, O.

Examples of monocyclic non-aromatic heterocyclic groups that can be envisaged are pyrrolidine, piperidine, tetrahydrofuran, tetrahydropyran, thiolane.

The term "monocyclic aromatic group" as used herein is referred to an aromatic group consisting only of one conjugated planar ring. Said monocyclic aromatic group can be heterocyclic.

Examples of monocyclic aromatic group likely to be used in the present invention include, but not limited to, phenyl, tolyl, phyridinyl, pyrazinyl, pyrimidinyl, pyridazynyl, pyrrolyl, thiophenyl, imidazolyl, oxazolyl, thiazolyl, pyrazolyl, furyl.

The term "(C₄-C₂₀)alkyl" is meant to a branched or unbranched saturated hydrocarbon group having from 4 to 20 carbon atoms. Examples of "(C₄-C₂₀)alkyl" can be, but not limited to, n-butyl, sec-butyl, iso-butyl, tert-butyl, n-pentyl, isopentyl, neopentyl, n-hexyl, n-heptyl, n-octyl, n-nonyl, n-decyl, n-undecyl, n-docenyl, n-tridecyl, n-tetradecyl, n-pentadecyl, n-hexadecyl, n-heptadecyl, n-octadecyl, n-nonadecyl, n-icosyl.

The term "no more than two of R₁, R2, R3 and R4 are hydrogen on the same time" means that the prodrug of formula I' contains at least two (C₄-C₂₀)alkyl chains which are identical or different.

In a preferred embodiment of the invention, A₁ of formula I' represents a linear or branched (C₁-C₄) alkyl comprising:
- at least one of 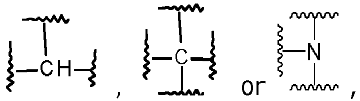
- one or more linker groups chosen from -NH-(C=O)-, -(C=O)-NH-,
- (C=O)O-, -(C=O)-, -O-(C=O)-NH-.

The term "(C₁-C₄)alkyl" is meant to a branched or unbranched saturated hydrocarbon group having from 1 to 4 carbon atoms. Examples of "(C₁-C₄)alkyl" can be, but not limited to methyl, ethyl, propyl, n-butyl, sec-butyl, iso-butyl, tert-butyl.

In another preferred embodiment of the invention, the prodrug is represent by the formula I' 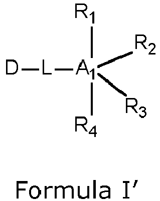 Wherein:
- R₁ and R₂ are identical or different and represent a (C₈-C₁₈)alkyl group;
- R3 and R4 are hydrogen;
- D, L, A₁ are as defined above.

In a more preferred embodiment of the invention, A₁ in the formula I' is a spacer

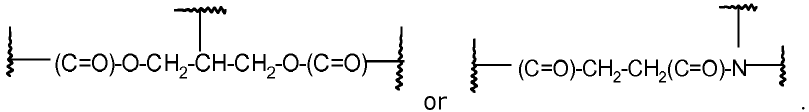

In a particularly preferred embodiment, the prodrug of the invention is represented by formula (I'a) or formula (I'b) 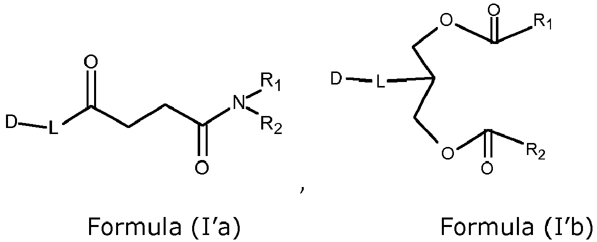 Wherein D, L, R₁ and R₂ are as defined above.

In a specific embodiment of the present invention, said prodrug is a prodrug of doxorubicin of formula (I'a1)

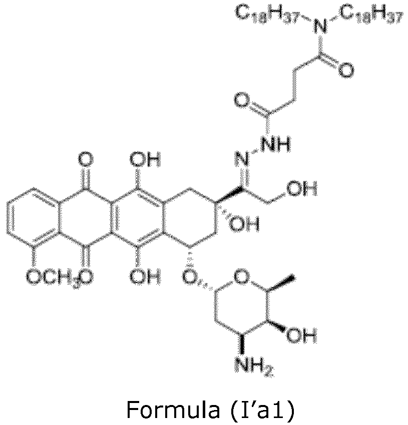

In another specific embodiment of the prodrugs of formula (I'a), said prodrug is one of the compounds below:

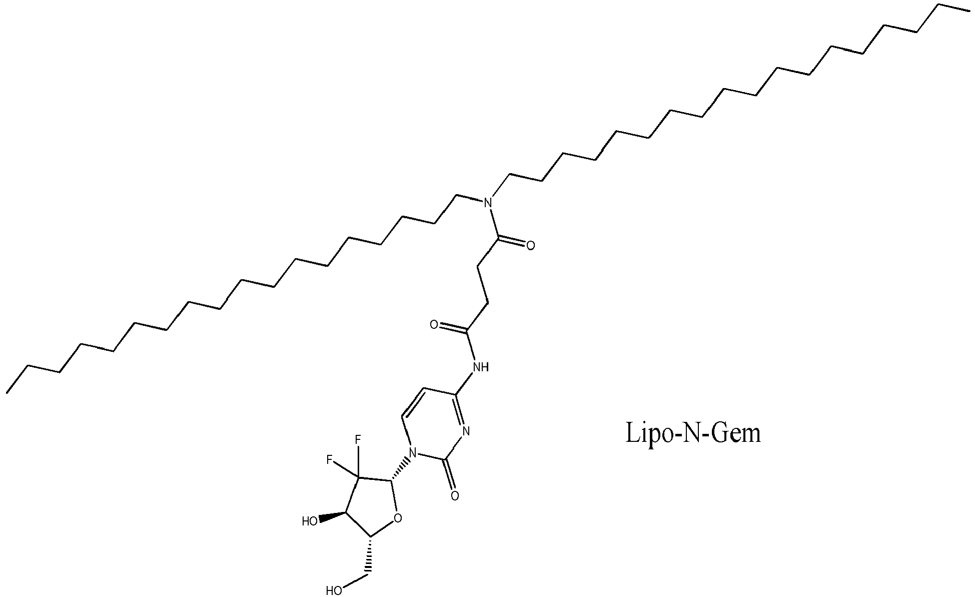

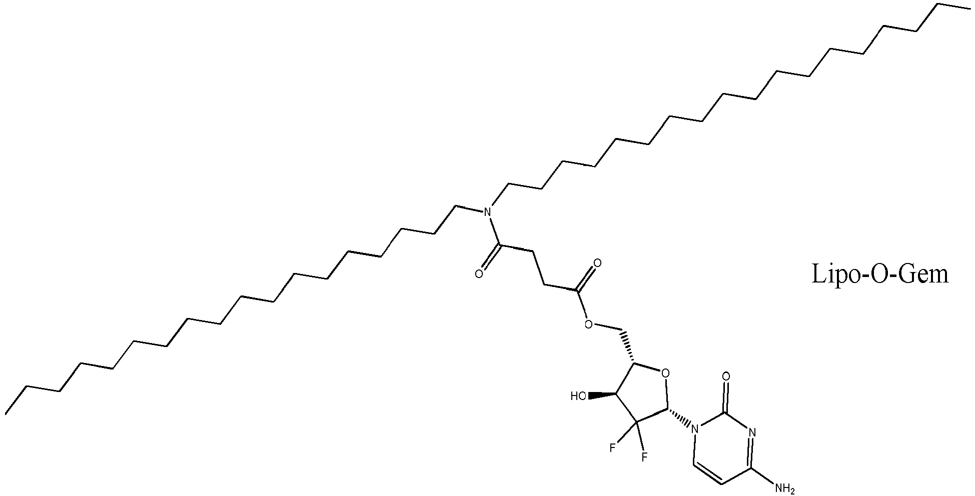

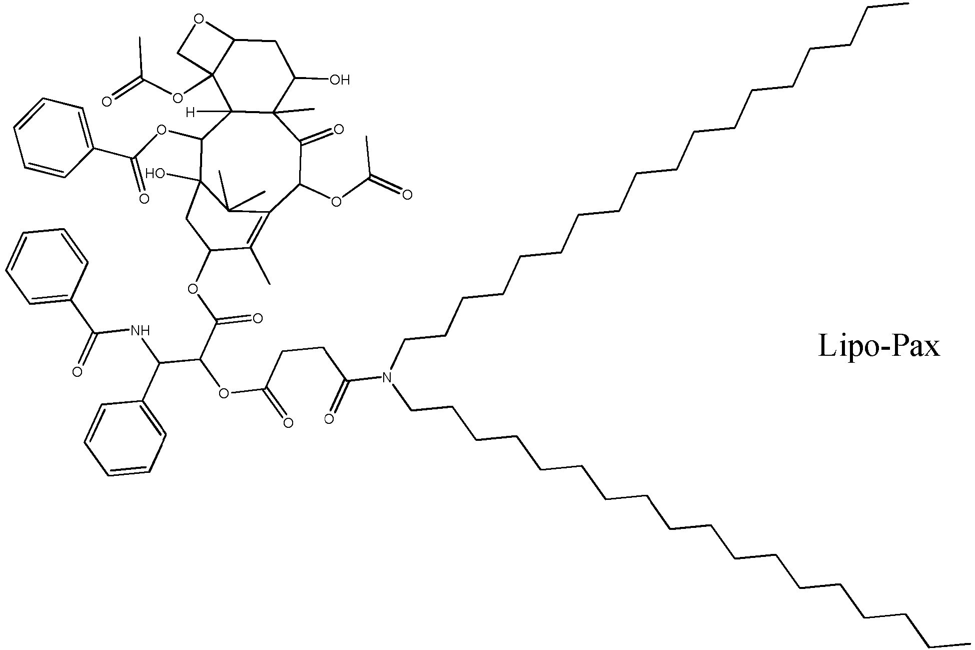

The compounds Lipo-N-Gem and Lipo-O-Gem are prodrugs of gemcitabine. The compound Lipo-Pax is the prodrug of paclitaxel.

According to a specific embodiment, the nanoemulsion described above of the present invention further comprises a surface functionalisation component of formula (III):

M-S-L-G Formula (III)

Wherein:
- M is a moiety, preferably a targeting ligand, having the propriety to functionalize said nanoemulsion,
- S is a hydrophilic spacer,
- L and G are as defined before for formula I.

Thanks to the presence of hydrophilic spacer S and of lipophilic moiety G, the surface functionalisation component of formula III is stably integrated in the nanoemulsion and the moiety M is exposed on the surface of nanoemulsion, which allows to functionalize said nanoemulsion.

Said hydrophilic spacer S can be a polyethylene glycol (PEG) chain of formula -(CH₂-CH₂-O)ₙ-, wherein n is an integer chosen from 1 to 300, or an oligopeptide.

The term "functionalize said nanoemulsion" used herein is meant to enable said nanoemulsion to be recognized by a target molecule, particularly a target cell, or enable said nanoemulsion to enter into a target cell.

Preferably, the moiety M is a targeting ligand which can be recognized by a targeted molecule, particularly a target cell.

Examples of moiety M include but not limited to, an antibody, a nanobody, an aptamer, an oligonucleotide, a peptide or small biologically active organic molecule.

In a particular embodiment, the nanoemulsions encapsulating a prodrug of formula I of the present invention is further be functionalized by antibodies, which enables said nanoemulsion to be recognized by target cells, such as a specific type of tumor.

The present invention concerns also a nanoemulsion encapsulating a prodrug of formula I as described above for its use as medicament.

The present invention concerns also a pharmaceutical composition comprising the nanoemulsion encapsulating a prodrug of formula I as described above.

Said pharmaceutical composition can further comprise a pharmaceutical acceptable carrier. As would be appreciated by one of skill in this art, the carriers may be chosen based on the route of administration as described below, the location of the target issue, the drug being delivered, the time course of delivery of the drug, etc.

Injectable preparations, for example, sterile injectable aqueous may be formulated according to the known art using suitable dispersing or wetting agents and suspending agents. The sterile injectable preparation may also be a sterile injectable solution, suspension, or emulsion in a nontoxic parentally acceptable diluent or solvent. Among the acceptable vehicles and solvents that may be employed are water, phosphate buffered saline, Ringer's solution, and isotonic sodium chloride solution.

Said pharmaceutical composition can be an injectable pharmaceutical composition comprising a prodrug of formula I which can release a therapeutically effective amount of a drug.

The therapeutically effective amount of said drug can be determined by common knowledge and conventional methods by a person skilled in drug development.

Another aspect of the present invention is to provide a nanoemulsion comprising a natural oil and/or a synthetic oil, and an amphiphilic surfactant, said nanoemulsion encapsulating:
- a lipophilic compound of formula II,

   E-G Formula II

   Wherein :
   - E represents a reactive group chosen from a hydrazide group, a hydrazine group, a O-substituted hydroxyl amine, a chloroformate group, a primary amine group, a secondary amine group, a carbonyl group, an active ester, an isocyanate group, an isothiocyanate group, a hydroxyl group,
   - G represents:
      (i) a derivative of sterol, whose polycyclic part is linked to the group E; or
      (ii) a group of formula A 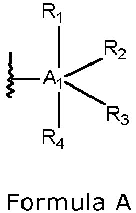 Wherein A₁ is a spacer chosen from:
         - a linear or branched alkyl chain comprising:
            ▪ from 1 to 8 methylene bridge,
            ▪ at least one 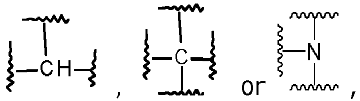
            ▪ optionally one or more linker groups chosen from -(C=O)-O-, -(C=O)-NH-, -O-(C=O)-NH-, -NH-, -(C=O)-, -O-;
         - a cyclo(C₃-C₇)alkyl eventually substituted by one or more linker groups chosen from -(C=O)-O-, -(C=O)-NH-, -O-(C=O)-NH-, -NH-, -(C=O)-, -O-, a monocyclic non-aromatic heterocyclic group eventually substituted by one or more linker groups chosen from -(C=O)-O-, -(C=O)-NH-, -O-(C=O)-NH-, -NH-, -(C=O)-, -O-, or a monocyclic aromatic group eventually substituted by one or more linker groups chosen from -(C=O)-O-, -(C=O)-NH-, -O-(C=O)-NH-, -NH-, -(C=O)-, -O-,
   - R₁ R₂, R₃, and R₄ are identical or different and each represent a (C₄-C₂₀)alkyl or a hydrogen, wherein no more than two of R₁, R₂, R₃ and R₄ are hydrogen on the same time.

The term "carbonyl" as used herein is meant to any aldehyde, ketone or carboxyl group.

The term "active ester" as used herein is meant to an ester capable to spontaneously react with amino and hydroxyl groups. Examples can be cited are N-hydroxysuccinimidyl active ester.

The use of compound of formula II brings several advantages. Firstly, the conjugation of this compound with a drug having only medium lipophilicity, such as doxorubicin, enables to improve lipophilicity of said drug by introducing into said drug an extremely lipophilic moiety G, and thus to prevent undesirable release of this drug from nanoemulsions.

Secondly, the compound of formula II, through its reactive group E, can form a biodegradable covalent bond with a drug bearing a keto group, an aldehyde group, a carboxyl group, a hydroxyl group, a primary amine group or a secondary amine group, thus make it possible to *in vivo* release the drug in a suitable physiological condition.

For example, hydrazone bond, which can be degraded in low pH condition (pH2 to 4), can be formed between a compound of formula II bearing a hydrazine group and a drug bearing a keto group or an aldehyde group.

Thirdly, formulation of nanoemulsion encapsulating highly lipophilic compound of formula II makes it possible to directly conjugate *in situ* said compound with a drug. This approach avoids prior synthesis of a prodrug in organic solvent that further needs purification and/or the detoxification of the medium from solvents, reactive reagents and ketone-containing drugs.

Thus, the nanoemulsions encapsulating said lipophilic compound of formula II can be used as a platform to directly *in situ* synthesize a prodrug inside the oily core of nanoemulsions.

In a specific embodiment, the nanoemulsion of the invention encapsulates a lipophilic compound of formula II' 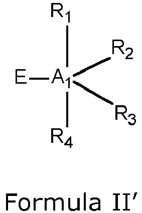 Wherein E, A₁, R₁ R₂, R₃, and R₄ are defined as before.

In a preferred embodiment of the invention, A₁ of formula II' represents a linear or branched (C₁-C₄) alkyl comprising:
- at least one of 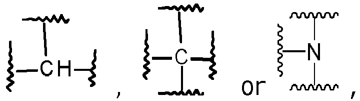
- one or more linker groups chosen from -NH-(C=O)-, -(C=O)-NH-, -(C=O)O-, -(C=O)-, -O-(C=O)-NH-.

According to another preferred embodiment of the invention, the lipophilic compound is represent by the formula II', wherein
- R₁ and R₂ are identical or different and represent a (C₈-C₁₈)alkyl group;
- R3 and R4 are hydrogen;
- E, A₁ are as defined above.

In a more preferred embodiment of the invention, A₁ in the formula II' is a spacer

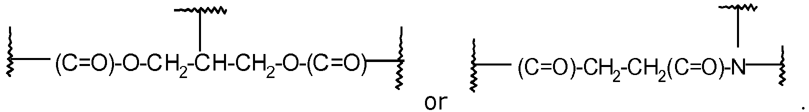

In a more specific embodiment, the nanoemulsion of the invention encapsulates a lipophilic compound of formula (II'a) or formula (II'b) 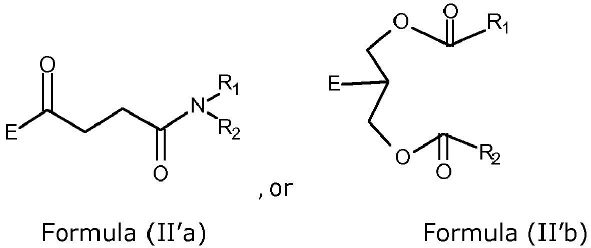 Wherein E, R₁, R₂ are defined as before.

Examples of a compound of formula II'a can be cited are

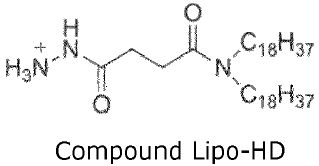

Nanoemulsions encapsulating a compound of formula II of the present invention can be produced by any conventional nanoemulsification methods, for example by spontaneous nanoemulsification (Anton et al., Pharm Res. 2011;28(5):978-85.; Mason et al., J. Phys.: Condens. Matter 2006; 18: R635-R666). According to said method, the compound of formula II is dissolved in oily phase, then the surfactant is added. After homogenisation, for example by magnetic stirring, the nanoemulsions are generated with the addition of water or an appropriate aqueous buffer, such as phosphate buffered saline.

In a particular embodiment of the present invention, the nanoemulsion comprising lipophilic compound of formula II further comprises a surface functionalisation component of formula III as described above:

M-S-L-G Formula (III)

The presence of surface functionalisation component of formula III allows to functionalize the nanoemulsion comprising lipophilic compound of formula II.

Said functionalized nanoemulsions provide a target-specified platform which can be used to directly *in situ* produce a functionalized nanoemulsion comprising a prodrug of formula I stably encapsulated inside the oily core of nanoemulsions.

The third aspect of the present invention is to provide a method for preparing a nanoemulsion encapsulating a prodrug, said nanoemulsion comprising a natural oil and/or a synthetic oil and an amphiphilic surfactant,
said prodrug being represented by formula I,

D-L-G Formula I

wherein:
- L is a biodegradable covalent bond chosen from a hydrazone, an imine, an ester, an amide, a carbamate, a carbonate, an acetal, a hemiaminal
- D is a moiety having pharmaceutical or cosmetic activity after being cleaved from the prodrug of formula I,
- G represents:
   (i) a derivative of sterol, whose polycyclic part is linked to the group E; or
   (ii) a group of formula A 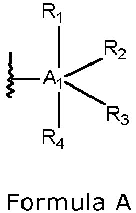 Wherein in A₁ is a spacer chosen from:
      ∘ a linear or branched alkyl chain comprising:
         ▪ from 1 to 8 methylene bridge,
         ▪ at least one 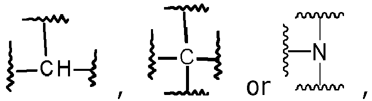
         ▪ optionally one or more linker groups chosen from -(C=O)-O-, -(C=O)-NH-, -O-(C=O)-NH-, -NH-, -(C=O)-, -O-;
      ∘ a cyclo(C₃-C₇)alkyl eventually substituted by one or more linker groups chosen from -(C=O)-O-, -(C=O)-NH-, -O-(C=O)-NH-, -NH-, -(C=O)-, -O-; a monocyclic non-aromatic heterocyclic group eventually substituted by one or more linker groups chosen from -(C=O)-O-, -(C=O)-NH-, -O-(C=O)-NH-, -NH-, -(C=O)-, -O-; or a monocyclic aromatic group eventually substituted by one or more linker groups chosen from -(C=O)-O-, -(C=O)-NH-, -O-(C=O)-NH-, -NH-, -(C=O)-, -O-,
- R₁, R₂, R₃, and R₄ are identical or different and each represent a (C4-C₂₀)alkyl or a hydrogen, wherein no more than two of R₁, R₂, R₃ and R₄ are hydrogen on the same time.
said method comprising the following step:
(a) reacting in a buffer a nanoemulsion encapsulating a lipophilic compound of formula II as described before with a drug bearing a keto group, an aldehyde group, a hydroxyl group, a primary amine group or secondary amine group, a carboxyl group, to obtain the nanoemulsion encapsulating a prodrug of formula I.

The step (a) of above-mentioned method can be carried out at a pH from 3 to 10 and at room temperature, especially from 15-25°C.

The buffer used in step (a) can be any conventional buffer which has a suitable pH value to favourite the reaction of step (a). Said buffer can be for example an acetate buffer of pH4.

The method of the present invention can also be applied to a drug which does not bear a group susceptible to react with the group E of the lipophilic compound of formula II. In this case, the method of the invention further comprises, before the step (a), a step to introduce into said drug a group which can react with the group E of the lipophilic compound of formula II, said group can be a keto group, an aldehyde group, a hydroxyl group, a primary amine group or secondary amine group, a carboxyl group.

The nanoemulsion encapsulating a prodrug of formula I according to the present invention can also be obtained by another method.

Said method comprises the following steps:
(a) reacting a drug bearing a keto group, an aldehyde group, a hydroxyl group, a primary amine group or secondary amine group, a carboxyl group with a lipophilic compound of formula II

   E-G Formula II

   Wherein :
   - E represents a reactive group chosen from a hydrazide group, hydrazine group, O-substituted hydroxyl amine, chloroformate group, a primary amine group, a secondary amine group, a carbonyl group, an active ester, an isocyanate group, isothiocyanate,a hydroxyl group,
   - G represents:
      (i) a derivative of sterol, whose polycyclic part is linked to the group E; or
      (ii) a group of formula A 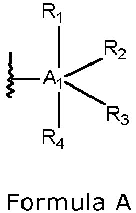 Wherein A1 is a spacer chosen from:
         ∘ a linear or branched alkyl chain comprising:
            ▪ from 1 to 8 methylene bridge,
            ▪ at least one 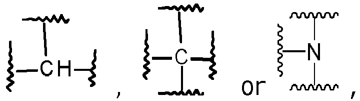
            ▪ optionally one or more linker groups chosen from -(C=O)-O-, -(C=O)-NH-, -O-(C=O)-NH-, -NH-, -(C=O)-, -O-;
         ∘ a cyclo(C₃-C₇)alkyl eventually substituted by one or more linker groups chosen from -(C=O)-O-, -(C=O)-NH-, -O-(C=O)-NH-, -NH-, -(C=O)-, -O-, a monocyclic non-aromatic heterocyclic group eventually substituted by one or more linker groups chosen from -(C=O)-O-, -(C=O)-NH-, -O-(C=O)-NH-, -NH-, -(C=O)-, -O-, or a monocyclic aromatic group eventually substituted by one or more linker groups chosen from -(C=O)-O-, -(C=O)-NH-, -O-(C=O)-NH-, -NH-, -(C=O)-, -O-,
   - R₁ R2, R3, and R4 are identical or different and each represent a (C4-C₂₀)alkyl or a hydrogen, wherein no more than two of R₁, R₂, R₃ and R₄ are hydrogen on the same time,
   to obtain a prodrug of formula I;
(b) mixing the prodrug of formula I, a natural oil and/or synthetic oil, and then with an amphiphilic surfactant to obtain a mixture;
(c) adding water to the mixture obtained in the step (b) to obtain a nanoemulsion encapsulating the prodrug of formula I.

The present invention is illustrated in more detail by following figures and examples.

### Figures

**Figure 1****:** Scheme of synthesis of DOX/Lipo-HD conjugate *in situ* inside lipid nanoemulsions.
**Figure 2A****:** Absorption spectra of 100 timed diluted nanoemulsions containing reference dye F888 and with Lipo-HD alone (F888-Lipo-HD), of 100 timed diluted nanoemulsions with Lipo-HD after incubation with DOX (F888-Lipo-HD-DOX), and of nanoemulsions encapsulating F888-Lipo-HD-DOX and further dialysis (F888-Lipo-HD-DOX after dialysis). Reference dye F888 was encapsulated at 0.5 wt% of with respect to Labrafac oil. Lipo-HD was encapsulated at 10 wt%.
**Figure 2B****:** Absorption spectra of 100 timed diluted nanoemulsions containing F888 but without Lipo-HD (F888), of 100 times diluted nanoemulsions containing F888 after incubation with DOX (F888-DOX), and of nanoemulsion encapsulating F888-DOX further dialysis (F888-DOX after dialysis). Reference dye F888 was encapsulated at 0.5 wt%.
**Figure 3A****:** Normalized absorption spectra of nanoemulsions with Lipo-HD containing F888 after incubation with DOX (F888-Lipo-HD-DOX) and further dialysis (F888-Lipo-HD-DOX (dialysis)). Reference dye F888 was encapsulated at 0.5 wt%. Lipo-HD was encapsulated at 10 wt%.
**Figure 3B****:** Normalized absorption spectra of nanoemulsions without Lipo-HD containing F888 (F888) and after incubation with DOX and further dialysis (F888-DOX (dialysis)). Reference dye F888 was encapsulated at 0.5 wt%.
**Figure 4A****:** Fluorescence spectra of nanoemulsions encapsulating 0.5 wt% of donor dye F888 with 10 wt% of Lipo-HD (F888-Lipo-HD) or without Lipo-HD (F888).
**Figure 4B****:** Fluorescence spectra of nanoemulsions with Lipo-HD incubated with DOX and then dialyzed (F888-Lipo-HD-DOX), of nanoemulsions without Lipo-HD incubated with DOX and then dialyzed (F888-DOX), and of nanoemulsions containing F888 alone (F888). All samples were diluted 300 times. All fluorescence spectra were corrected from the blank.
**Figures 5A, 5B****:** Fluorescence spectra of F888-Lipo-HD-DOX nanoemulsions (5000-fold dilution) in the presence of blank nanoemulsions (50-fold dilution) at pH 7.4 (Fig. 5A) and 4.0 (Fig. 5B). Excitation wavelength was 390 nm. All fluorescence spectra were corrected from the blank.
**Figures 5C****:** Fluorescence intensity ratio donor/acceptor as a function of incubation time for pH 7.4 and 4.0 (Fig. 5C).
**Figure 6A, 6B****:** Absorption spectra of four nanoemulsions containing respectively a compound of formula I'a, where R1 and R2 are butyl (C4)-alkyl chains, or octyl (C8)-alkyl chains, or octadecyl (C18)-alkyl chains, or R1 is a single C18 alkyl-chain, while R2 = H (C18 mono chain). The measurement was done on a freshly prepared nanoemulsion (6A) or after 4 days storage at 4°C (6B).
**Figure 7****:** Photo of four nanoemulsions obtained by the reaction of DOX with respectively a compound of formula I'a bearing two butyl (C4)-alkyl chains, or two octyl (C8)-alkyl chains, or two octadecyl (C18)-alkyl chains or one single C18 alkyl-chain. White circle shows the presence of precipitate in case of derivative with single chain.
**Figure 8****:** Cytotoxicity of different cell lines (MDA-MB-231, Jimt1 and 4T1 cells) after incubation for 48h with Lipo-HD-DOX loaded nanoemulsion or free DOX drug at different concentrations of the drug.
**Figure 9****:** Cytotoxicity of different cell lines (MDA-MB-231, Jimt1 and 4T1 cells) after incubation for 48h with Lipo-PAX and Lipo-N-Gem loaded nanoemulsion or corresponding free drugs at different drug concentrations.

### Examples

### Example 1:

### Materials and methods

### 1. Synthesis of derivatives of compound Lipo-HD.

**4-(dioctadecylamino)-4-oxobutanoic acid (1).** To the solution of succinic anhydride (2.61 g, 5 mmol) in DCM (20 mL) was added DMAP (5 mg) and dioctadecylamine (0.50 g, 5 mmol), then mixture was stirred at R.T. overnight under argon atmosphere. The solvent was evaporated in vacuo, then resulting residue was purified by recrystallization from EtOH to give desired product as a colorless solid (2.65 g, 85%). 1H NMR (400 MHz, CDCl3) δ ppm 11.72 (1H, bs), 3.34 (4H, t), 3.19 (4H, t), 1.56 (4H, bs), 1.24 (60H, bs), 0.86 (6H, t). MS for [C40H79NO3 - H⁺]⁻ m/z (M-1) calc. 620.61, found 620.60.

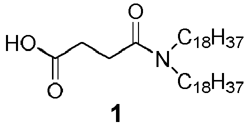

**Tert-butyl 2-(4-(dioctadecylamino)-4-oxobutanoyl)hydrazinecarboxylate (2).** To the solution of compound 1 (2.0 g, 3.22 mmol) in DCM (50 mL), EDC-HCl (2.0 g, 7.07 (2.2 eq)) and tert-butylcarbazate (0.85 g 6.43 mmol (2.0 eq)) were added and stirred at room temperature overnight under argon atmosphere. The mixture was washed by water and brine, and then organic phase was dried over by MgSO4. The solvent was evaporated in vacuo, then resulting residue was purified by flash column chromatography on silica gel (eluent EtOAc/Hepane 50:50) to give desired product as a colourless solid (2.16 g, yield 91%). 1H NMR (400 MHz, CDCI3) δ ppm 8.14 (1H, bs), 6.36 (1H, bs), 3.29 (2H, t), 3.21 (2H, t), 2.68 (2H, t), 2.58 (2H, t), 1.53 (13H, bs), 1.20 (60H, bs), 0.88 (6H, t). MS for [C45H89N3O4+H⁺]⁺ m/z (M+1) calc. 736.69, found 736.69.

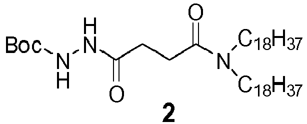

**Lipo-HD.** Compound 2 (1 eq., 430 mg, 0.584 mmol) was dissolved in 10 ml of dry DCM and 1 ml of TFA was added. The mixture was left to stir for 2h and then washed with 10% aqueous NaHCO3. DCM layer was dried over sodium sulphate, filtered and evaporated. The crude product was purified by silica gel chromatography (DCM/MeOH 99/1 - 80/20) to obtain 3-(hydrazinecarbonyl)-N,N-dioctadecylpropanamide (140 mg, 0.22 mmol, 38 %). 1H NMR (400 MHz, CDCI3) δ ppm 7.77 (1H, bs), 3.76 (2H, bs), 3.27 (2H, t), 3.16 (2H, t), 2.65 (2H, t), 2.49 (2H, t), 1.50 (4H, bd), 1.24 (60H, bs), 0.84 (6H, t). MS for Chemical Formula: [C40H81N3O2+H] m/z (M+1) calc. 636.63, found 636.63.

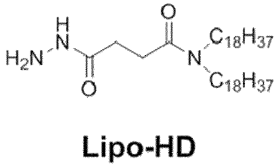

### 2. Synthesis of DOX/Lipo-HD conjugate in situ inside lipid nanoemulsions

The compound Lipo-HD was dissolved firstly into the oil Labrafac® at 10 wt%. Then 65 mg of surfactant Solutol was added to 35 mg of above solution. The mixture was homogenized under magnetic stirring at 90°C for 10 min up to complete homogenisation. Finally, nanoemulsions were generated with the addition of ultrapure (Milli-Q) water (230 mg). The size of nanoemulsions could be monitored by the variation of the ratio between oil (labrafac) and surfactant (solutol).

For fluorescence studies, 0.5 wt% of a donor dye F888 is added respectively to Labrafac® containing or not 10 wt% of Lipo-HD. Then the nanoemulsions were prepared as described above. F888 was used for monitoring the encapsulation, release and stability of the nanoemulsion by looking on the intensity variation between F888 and DOX. The chemical structure of compound F888 is given below (Klymchenko et al., RSC Adv., 2012, 2(31): 11876-11886):

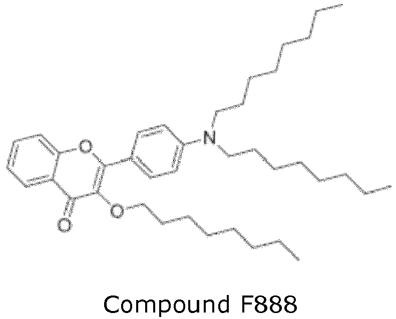

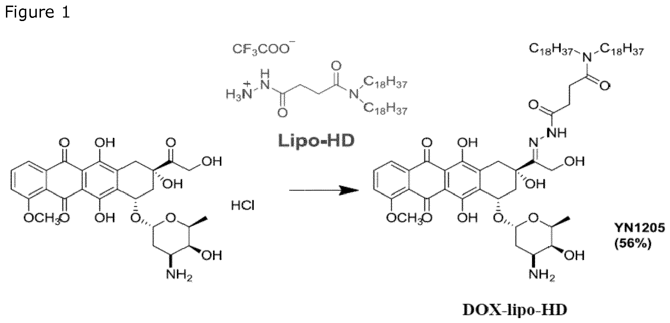

Those initial solutions were 10-fold diluted in 1 mL of 20 mM acetate buffer (pH 4). To those solutions, 50 mL of DOX-HCl (30 mM) was added. The mixtures were vortexes for 12 h at 37 °C. The Scheme of synthesis is illustrated in figure 1.

The resulting solutions were dialyzed in 500 mL of 0.1M NaHCO3 for 12h.

In total, 4 formulations of nanoemulsions were prepared and analysed in the following experiments:
- F888-Lipo-HD (100 dilu)
- F888-Lipo-HD-DOX (100 dilu)
- F888 (100 dilu)
- F888-DOX (100 dilu)

Size distributions were determined by dynamic light scattering using Zetasizer Nano ZSP (Malvern Instruments S.A).

### 3. Spectroscopic absorption of nanoemulsions

Absorption spectra were recorded on a Cary® spectrophotometer (Varian) according to conventional measurement method.

### 4. Stability of loading and pH-controlled release

FRET technique allows monitoring the leakage of loaded dyes, because this process separate donor from acceptor (Klymchenko et al, RSC Adv., 2012, 2(31):11876-11886). To model dye leakage, the dye-loaded nanoemulsion was mixed with 100-fold excess of blank particles, which acted as the acceptor medium for the released dyes.

Fluorescence spectra were recorded by a Fluorologue (Jobin Yvon, Horiba) spectrofluoremeter. Fluorescence emission spectra were performed at room temperature with 390 excitation wavelengths for the donor F888.

### Results

### Characterization of encapsulation of DOX-Lipo-HD into nanoemulsions

The size of formulated nanoemulsions (before the dialysis) remained stable and independent of the loaded compounds (Table 1).

**Table 1. Size measurements of formulated nano-droplets (before dialysis)**

| Sample | Diameter (nm) | Polydispersity index (PDI) |
|---|---|---|
| F888 0.5 wt%-Lipo-HD 10 wt% | 28.26 | 0.069 |
| F888 0.5 wt% | 36.56 | 0.068 |
| F888 0.5 wt%-Lipo-HD 10 wt%-DOX | 31.36 | 0.07 |

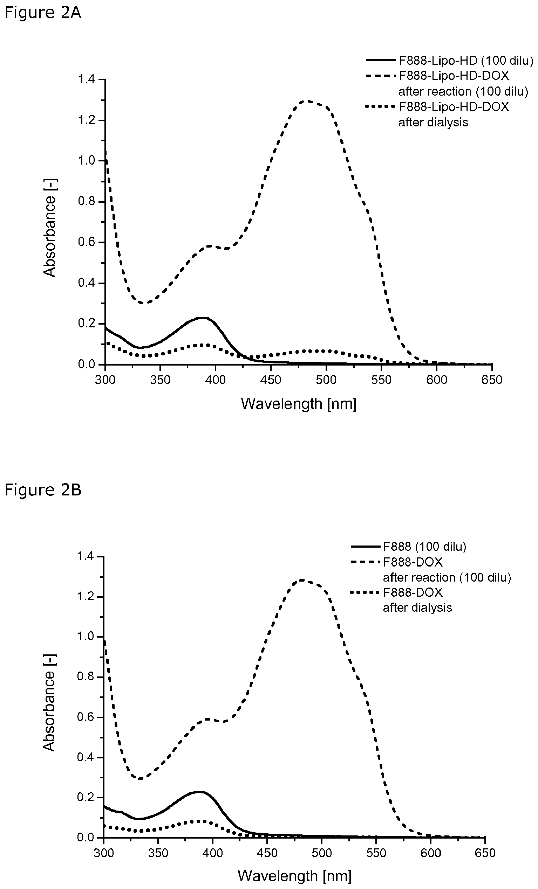

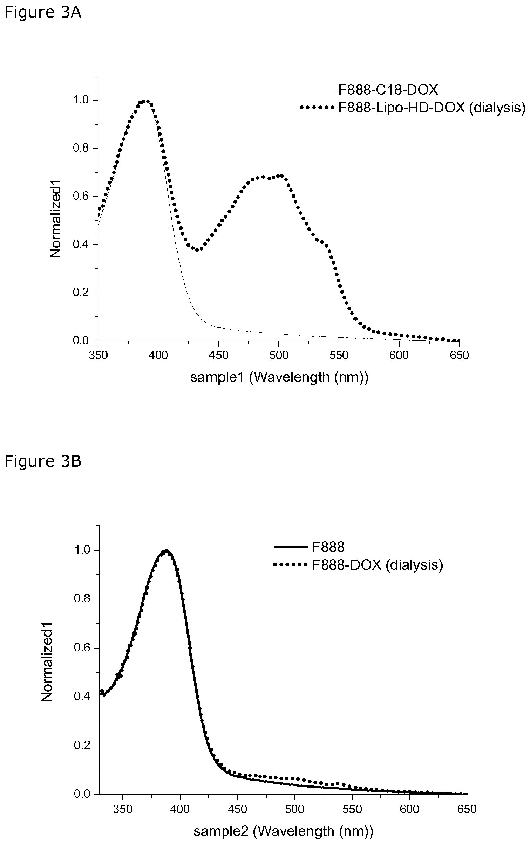

In the absorption spectra before the dialysis, strong absorption band of DOX was observed for both formulations with and without Lipo-HD. However, after dialysis, the formulation without Lipo-HD totally lost absorption of DOX, while for that containing Lipo-HD a significant absorption of DOX was still observed (Fig. 2A, 2B). Normalized absorption spectra (Fig. 3A, 3B) helped to compare absorbance with the reference compound F888. Samples containing Lipo-HD showed 60% of absorbance of DOX with respect to F888, while for those without Lipo-HD, the absorbance of DOX was negligibly low. Based on the extinction coefficient of DOX 12 200 M⁻¹cm⁻¹ (Bromberg et al., Langmuir, 2002, 18 (12): 4944-4952), the loading could be calculated as 1.6 wt% with respect to labrafac ® oil. Without Lipo-HD, the loading was <0.06% and could be neglected.

It is observed that Lipo-HD-Dox can be formed in situ after addition of DOX to nanoemulsions containing Lipo-HD. The experimental results (Fig. 2A, 2B, 3A, 3B) show that Lipo-HD-Dox is much better soluble in oil than DOX alone and that Lipo-HD-Dox remain inside nanoemulsions after dialysis, while DOX alone is leaked out from the nanoemulsions containing DOX.

### FRET-based evidence for encapsulation and pH-controlled release

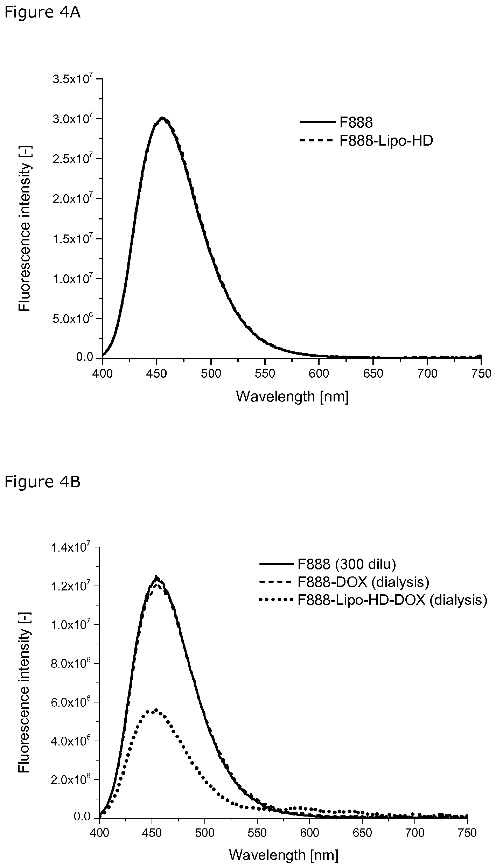

Fluorescence spectra of the nanoemulsions were studied. The fluorescence intensity of the reference dye F888 was not affected by the presence of 10 wt% of Lipo-HD (Fig. 4A). On the other hand, when nanoemulsions containing F888 and Lipo-HD were incubated with DOX and further dialyzed, the fluorescence intensity of F888 decreased and a shoulder around 580-590 nm appeared (Fig 4B). This result indicates the presence of FRET from F888 to DOX, which is known to emit around 560-590 nm (Motlagh et al., Biomed Opt Express. 2016 Jun 1; 7(6): 2400-2406). However, the shoulder was weak, which can be explained by much lower fluorescence quantum yield of DOX (7.1% in ethanol) compared to F888 (∼70% in nanoemulsion). By contrast, the incubation of nanoemulsions without Lipo-HD with DOX and further dialysis gave nanoemulsions without any FRET signal, as the emission of F888 was the same as control nanoemulsions (containing only F888) and no shoulder at 580-590 nm was observed. Thus, fluorescence data confirm the successful encapsulation of DOX and suggest the presence of DOX inside the oil core together with the reference dye F888.

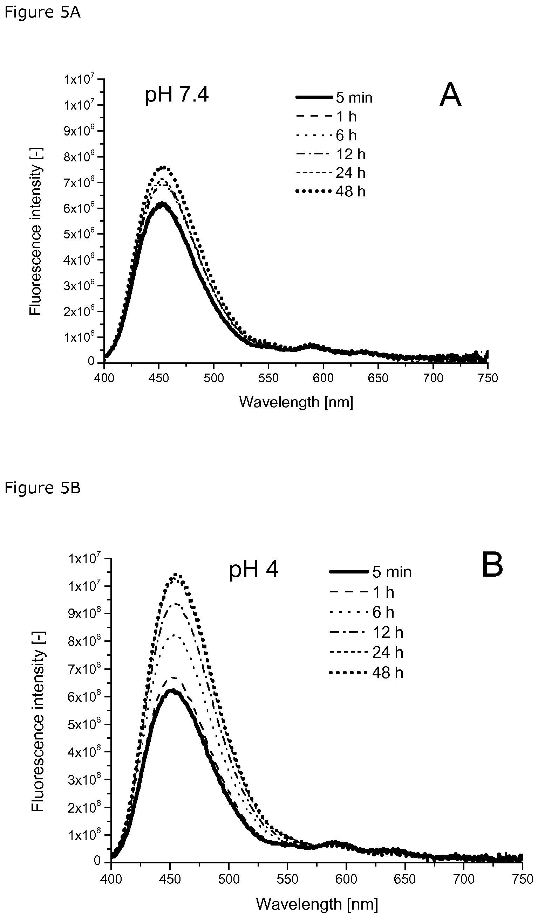

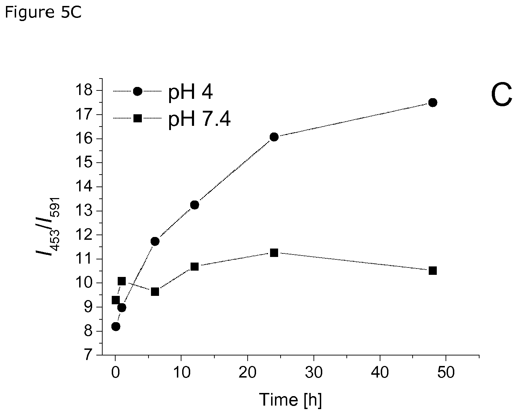

To monitor the pH-controlled release of DOX, FRET technique was used. The final F888/Lipo-HD/DOX nanoemulsions were mixed with 100-fold excess of blank nanoemulsions and the emission spectra were recorded as a function of time at pH 7.4 (phosphate buffer) and 4.0 (acetate buffer). At pH 7.4, minimal changes of the spectra were observed (Fig. 5A), so that there was nearly no change in the ratio donor /acceptor (Fig. 5C), suggesting no release of DOX at pH 7.4. By contrast at pH 4, gradual changes in the spectra were observed (Fig. 5B), so that the donor/acceptor intensity ratio increased over time (Fig. 5C). These changes suggest the release of DOX is probably due to hydrolysis of the hydrazone at pH 4.0. In conclusion, with help of Lipo-HD, DOX can be encapsulated efficiently inside nanoemulsions and can be released under pH control (at low pH). The results of the present invention illustrate the application of nanoemulsions with Lipo-HD as carrier of anti-cancer drug DOX that can be released specifically in acidic compartments of cancer tissues.

### Example 2:

### • Nanoemulsions preparation

Three compounds of formula II'a, analogues of Lipo-HD, having respectively two C4-alkyl chains (Lipo-HD-diC4), or two C8-alkyl chains (Lipo-HD-diC8), or one single C18 alkyl-chain (Lipo-HD-C18), were synthesized by similar protocols as described for Lipo-HD in Example 1. These three compounds and Lipo-HD were used in nanoemulsion formulation.

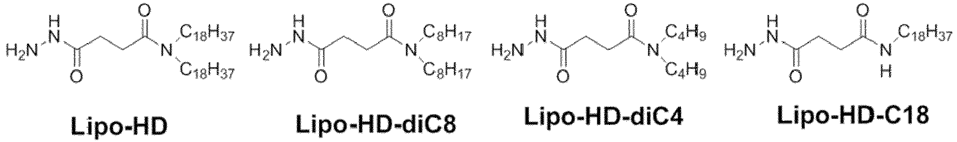

Corresponding compounds of formula II'a were respectively dissolved first into the oil Labrafac® at 5 wt%. Then 45 mg of surfactant Cremophore ® was added to 55 mg of above solution. The mixture was homogenized under magnetic stirring at 40°C for 10 min up to complete homogenisation. Finally, nanoemulsions were generated with the addition of phosphate buffered saline (230 mg).

300 µl of the obtained nanoemulsion encapsulating the compound of formula II'a were diluted into 300 µl of 20 mM acetate buffer (pH=4), and then 50 µl of 30 mM solution of doxorubicin in DMSO was added. The mixture was reacted under shaking at 37°C during 24h.

After the reaction, the sample was dialysed for 24h at NaHCO₃ buffer (0.1 M) at pH8 and then centrifugation at 6000 rpm for 1 minute was carried out.

### • Results

The proprieties of those four nanoemulsions were characterised and illustrated in Table 2 below.

**Table 2**

| | **Lipo-HD-diC4** | **Lipo-HD-diC8** | **Lipo-HD** | **Lipo-HD-C18** |
|---|---|---|---|---|
| **loading efficiency (%)** | 20% | 49% | 55% | 21% |
| **Encapsulation loading (%)** | 0.64% | 1.56% | 1.58% | 0.68% |
| **Size (nm)** | 106nm | 95nm | 99nm | 104nm |
| **PDI** | 0.115 | 0.116 | 0.118 | 0.132 |

The compound Lipo-HD-Dox having two C18-alkyl chains (Lipo-HD) give the best result for loading efficiency and encapsulation loading (Table 2). The achieved loading for Lipo-HD is significantly higher than the analogue bearing a single octadecyl chain.

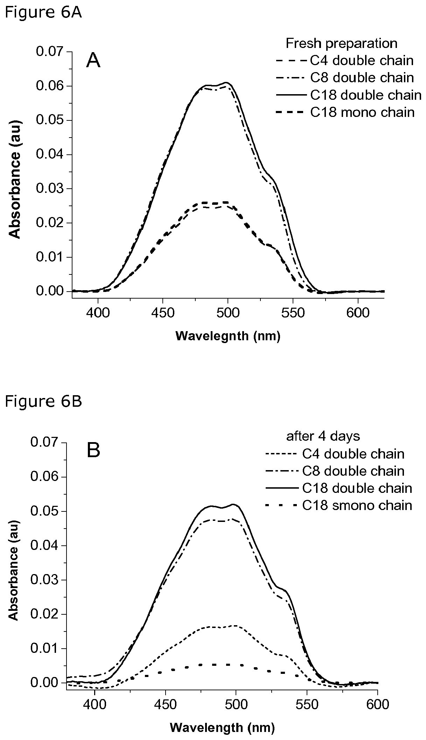

Absorption spectra showed that after 4 days on storage of these four nanoemulsions, the compounds containing C8 (Lipo-HD-diC8) and C18 (Lipo-HD) double chains showed minimal changes, while for C18 mono chain (Lipo-HD-C18), a strong loss of absorbance was observed (Figure 6A and 6B). This result showed that mono chain derivative does not provide sufficient stability to nanoemulsion, while doxorubicin derivatives with two aliphatic chains are rather stable at the same conditions.

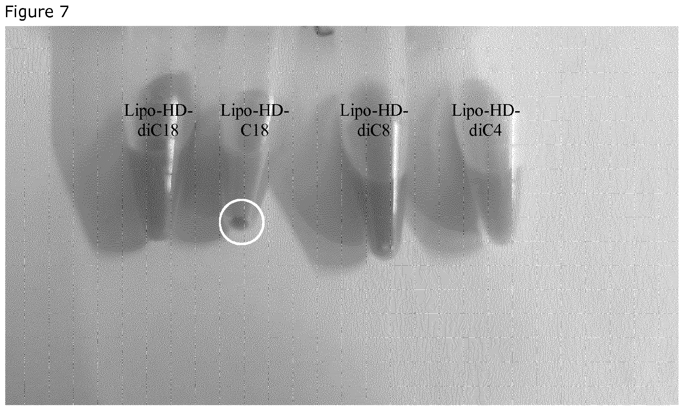

Indeed, it can be observed in figure 7 that nanoemulsions containing a compound of Lipo-HD-C18 with DOX having a single C18-alkyl chain shows signs of precipitation already 4 days after preparation.

### Example 3: In vitro toxicity evaluation of Lipo-HD-DOX

### Materials and methods

### MTS cytotoxicity assay in cell culture.

Three mammary carcinoma cell lines were used (mouse origin: 4T1, Human origin: MDA-MB-231 and Jimt1). The MTS assay is based on the reduction of the MTS tetrazolium compound by viable mammalian cells (here, by tumor cells) to generate a colored formazan dye that is soluble in cell culture media. The formazan dye is quantified by measuring the absorbance at 490-500 nm. Briefly, tumor cells (MDA-MB-231, Jimt1 or 4T1 cells) were seeded (5000) in a 96 wells plate. After 24h, the medium was removed and replaced by drug-containing (LipoHD-DOX nanoemulsion or DOX alone) medium. After 48h, the medium was removed and replaced with MTS-containing medium (0,5 mg/ml). After 4h of incubation, the medium was removed, replaced with 100µl of DMSO (agitation, 20 min) and the absorbance was read at 570 nm.

### Results

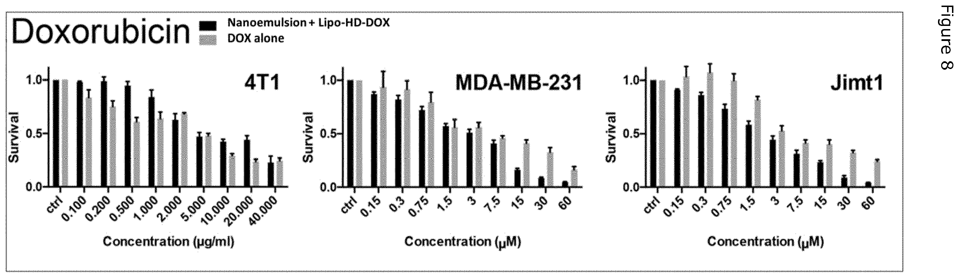

The effect of prodrug-loaded (Lipo-HD-DOX) nanoemulsions has been tested on three different cell lines (MDA-MB-231, Jimt1 or 4T1 cells) using the classical MTS assay, which is a method allowing sensitive quantification of viable cells and thus of cytotoxicity. As shown in Fig. 8, increasing concentrations of doxorubicin alone (without nanoemulsion) led to a dose-dependent cytotoxicity. Interestingly, NE-encapsulated prodrug Lipo-HD-DOX behaved either similarly, or with increased efficiency. The higher toxicity of the prodrug-loaded nanoemulsion compared to the free drug was observed for MDA-MB-231 and Jimt1 cells. Thus, Lipo-HD-DOX is probably metabolized by the cells liberating the free drug. The higher toxicity could result from more prolonged liberation of the drug, controlled by the cleavage of the hydrazone bond.

### Example 4: In vitro toxicity evaluation of prodrugs Lipo-PAX and Lipo-N-Gem in nanoemulsions

Lipo-PAX and Lipo-N-Gem were formulated with nanoemulsions using similar protocol as described in example 2. Corresponding prodrugs Lipo-PAX and Lipo-N-Gem were respectively dissolved first into the oil Labrafac® at 15 and 20 wt%, respectively. Then 45 mg of surfactant Cremophore ® was added to 55 mg of above solution. The mixture was homogenized under magnetic stirring at 40°C for 10 min up to complete homogenisation. Finally, nanoemulsions were generated with the addition of phosphate buffered saline (230 mg).

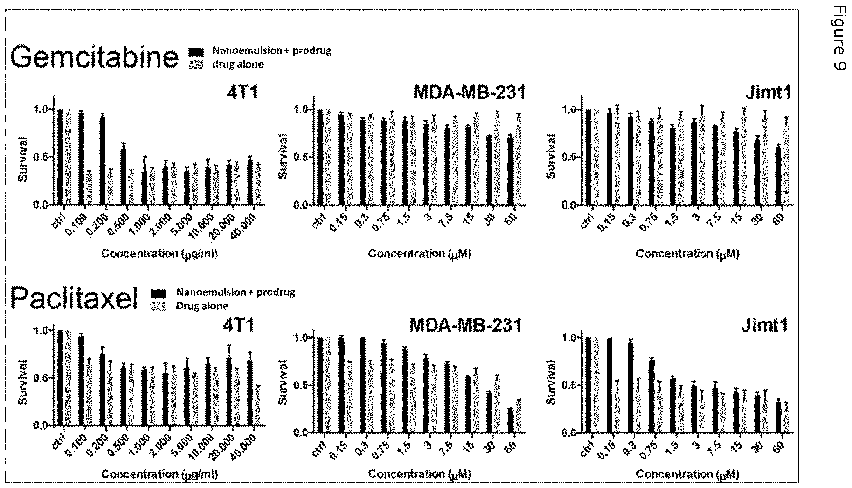

In vitro toxicity of prodrugs of paclitacel (Lipo-PAX) and gemcitabine (Lipo-N-Gem) in nanoemulsion were evaluated according to the method described before in example 3 and compared to corresponding free drugs. As shown in Figure 9, the cytotoxicity of free drug and corresponding prodrugs in nanoemulsions is dose-dependent, but their cytotoxicity is slightly different. In case of paclitacel, the cytotoxicity of drug alone is slightly higher than that of Lipo-PAX in the nanoemulsion, while Lipo-N-Gem in nanoemulsions is slightly more efficient that the free drug for MD-MB-231 and Jimt1 cells. Overall, it can be concluded that both prodrugs in nanoemulsions preserved their toxicity, which is probably because they can liberate the free drug after being metabolized by the cells (due to cleavage of the amide/ester bond). This is important for their therapeutic applications *in vivo.*

## Claims

1. A nanoemulsion encapsulating a prodrug, said nanoemulsion comprising a natural oil and/or a synthetic oil and an amphiphilic surfactant, said prodrug being represented by formula I,
D-L-G Formula I
wherein:
- L is a biodegradable covalent bond chosen from a hydrazone, an imine, an ester, an amide, a carbamate, a carbonate, an acetal, a hemiaminal
- D is a moiety having pharmaceutical, cosmetic or biological activity,
- G represents:
(i) a derivative of sterol, whose polycyclic part is linked to the group L; or
(ii) a group of formula A 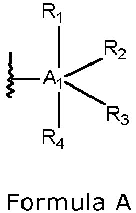 Wherein in A1 is a spacer chosen from:
∘ a linear or branched alkyl chain comprising:
▪ from 1 to 8 methylene bridge, and
▪ at least one 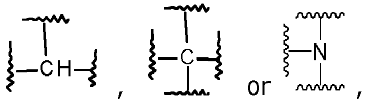
▪ optionally one or more linker groups chosen from -(C=O)-O-, -(C=O)-NH-, -O-(C=O)-NH-, -NH-, -(C=O)-, -O-;
∘ a cyclo(C₃-C₇)alkyl eventually substituted by one or more linker groups chosen from -(C=O)-O-, -(C=O)-NH-, -O-(C=O)-NH-, -NH-, -(C=O)-, -O-; a monocyclic non-aromatic heterocyclic group eventually substituted by one or more linker groups chosen from -(C=O)-O-, -(C=O)-NH-, -O-(C=O)-NH-, -NH-, -(C=O)-, -O-; or a monocyclic aromatic group eventually substituted by one or more linker groups chosen from -(C=O)-O-, -(C=O)-NH-, -O-(C=O)-NH-, -NH-, -(C=O)-, -O-,
- R₁, R₂, R₃, and R₄ are identical or different and each represent a (C4-C₂₀)alkyl or a hydrogen, wherein no more than two of R₁, R₂, R₃ and R₄ are hydrogen on the same time.

2. The nanoemulsion according to claim 1, encapsulating a prodrug of formula I' 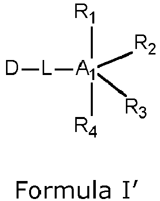 Wherein D, L, A₁, R₁, R₂, R₃ and R₄ are defined as claim 1.

3. The nanoemulsion according to claim 1 or 2, wherein A₁ represents a linear or branched (C₁-C₄) alkyl comprising:
- at least one of 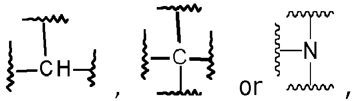
- one or more linker groups chosen from -NH-(C=O)-, -(C=O)-NH-, -(C=O)-, -(C=O)O-, -O-(C=O)-NH-.

4. The nanoemulsion according to claims 2 or 3, wherein A₁ in the formula I' is a spacer

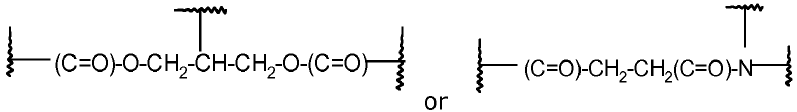

5. The nanoemulsion according to any one of claims 2 to 4, wherein the prodrug is represented by the formula (I'a) or formula (I'b) 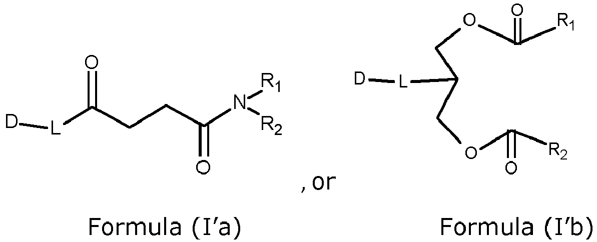 Wherein D, L, R₁ and R₂ are defined as claim 1.

6. The nanoemulsion according to any one of claims 1 to 5, wherein the diameter of the nanoemulsion is from 20 to 300 nm.

7. The nanoemulsion according to any one of claims 1 to 6, wherein the prodrug is a prodrug of an anticancer drug, in particular doxorubicin, paclitaxel, gemcitabine, rucaparib, docetaxel, decitabine, epirubicine, capecitabine, capecitabine, bendamustine, palbociclib, cytarabine, dabrafenib (GSK2118436), ribociclib, topotecan hcl, niraparib, ribociclib, lorlatinib, daunorubicin HCl, ibrutinib, mitomycin C, niraparib, methotrexate, amonafide, belizatinib, apitolisib (GDC-0980, RG7422), NU1025.

8. The nanoemulsion according to any one of claims 1 to 7, further comprising a surface functionalisation component of formula (III):
M-S-L-G Formula (III)
Wherein:
M is a moiety, preferably a targeting ligand, having the propriety to functionalize said nanoemulsion,
S is a hydrophilic spacer,
L and G are as defined in claim 1.

9. The nanoemulsion according to any one of claims 1 to 8, for its use as medicament.

10. A pharmaceutical composition comprising the nanoemulsion according to any one of claims 1 to 9.

11. A nanoemulsion comprising a natural and/or synthetic oil, and an amphiphilic surfactant, said nanoemulsion encapsulating :
- a lipophilic compound of formula II,
E-G Formula II
Wherein :
- E represents a reactive group chosen from a hydrazide group, hydrazine group, O-substituted hydroxyl amine, chloroformate group, a primary amine group, a secondary amine group, a carbonyl group, an active ester, an isocyanate group, isothiocyanate, a hydroxyl group,
- G represents:
(i) a derivative of sterol, whose polycyclic part is linked to the group E; or
(ii) a group of formula A 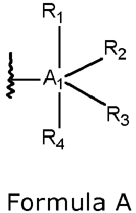 Wherein A1 is a spacer chosen from:
- a linear or branched alkyl chain comprising:
▪ from 1 to 8 methylene bridge, and
▪ at least one 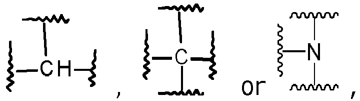
▪ optionally one or more linker groups chosen from -(C=O)-O-, -(C=O)-NH-, -O-(C=O)-NH-, -NH-, -(C=O)-, -O-;
- a cyclo(C₃-C₇)alkyl eventually substituted by one or more linker groups chosen from -(C=O)-O-, -(C=O)-NH-, -O-(C=O)-NH-, -NH-, -(C=O)-, -O-, a monocyclic non-aromatic heterocyclic group eventually substituted by one or more linker groups chosen from -(C=O)-O-, -(C=O)-NH-, -O-(C=O)-NH-, -NH-, -(C=O)-, -O-, or a monocyclic aromatic group eventually substituted by one or more linker groups chosen from -(C=O)-O-, -(C=O)-NH-, -O-(C=O)-NH-, -NH-, -(C=O)-, -O-,
- R₁ R₂, R₃, and R₄ are identical or different and each represent a (C4-C₂₀)alkyl or a hydrogen, wherein no more than two of R₁, R₂, R₃ and R₄ are hydrogen on the same time.

12. The nanoemulsion according to claim 11, encapsulating a lipophilic compound of formula II' 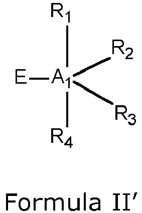 Wherein E, A₁, R₁ R₂, R₃, and R₄ are defined as claim 11.

13. The nanoemulsion according to claim 11 or 12, wherein the lipophilic compound is chosen from the compound of formula (II'a) or formula (II'b) 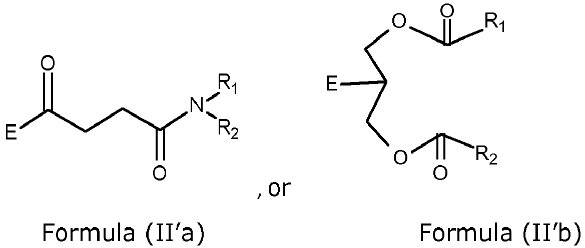 Wherein E, R₁, R₂ are defined as claim 11.

14. The nanoemulsion according to one of claims 11 to 13, further comprising a surface functionalisation component of formula (III):
M-S-L-G Formula (III)
Wherein:
- M is a moiety, preferably a targeting ligand, having the propriety to functionalize said nanoemulsion,
- S is a hydrophilic spacer,
- L and G are as defined in claim 1.

15. A method for preparing a nanoemulsion encapsulating a prodrug, said nanoemulsion comprising a vegetal or synthetic oil and an amphiphilic surfactant, said prodrug being represented by formula I,
D-L-G Formula I
wherein:
- L is a biodegradable covalent bond chosen from a hydrazone, an imine, an ester, an amide, a carbamate, a carbonate, an acetal, a hemiaminal
- D is a moiety having pharmaceutical or cosmetic activity after being cleaved from the prodrug of formula I,
- G represents:
(i) a derivative of sterol, whose polycyclic part is linked to the group E; or
(ii) a group of formula A 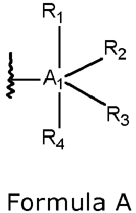 Wherein in A1 is a spacer chosen from:
∘ a linear or branched alkyl chain comprising:
▪ from 1 to 8 methylene bridge, and
▪ at least one 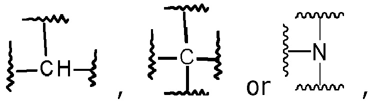
▪ optionally one or more linker groups chosen from -(C=O)-O-, -(C=O)-NH-, -O-(C=O)-NH-, -NH-, -(C=O)-, -O-;
∘ a cyclo(C₃-C₇)alkyl eventually substituted by one or more linker groups chosen from -(C=O)-O-, -(C=O)-NH-, -O-(C=O)-NH-, -NH-, -(C=O)-, -O-, a monocyclic non-aromatic heterocyclic group eventually substituted by one or more linker groups chosen from -(C=O)-O-, -(C=O)-NH-, -O-(C=O)-NH-, -NH-, -(C=O)-, -O-, or a monocyclic aromatic group eventually substituted by one or more linker groups chosen from -(C=O)-O-, -(C=O)-NH-, -O-(C=O)-NH-, -NH-, -(C=O)-, -O-,
- R₁, R₂, R₃, and R₄ are identical or different and each represent a (C4-C₂₀)alkyl or a hydrogen, wherein no more than two of R₁, R₂, R₃ and R₄ are hydrogen on the same time.
said method comprising the following step:
(a) reacting in a buffer a nanoemulsion as described in claims 11 to 14 with a drug bearing a keto group, an aldehyde group, a hydroxyl group, a primary amine group or secondary amine group, a carboxyl group, to obtain the nanoemulsion encapsulating a prodrug of formula II.

16. The method according to claim 15, further comprising, before the step (a), a step to introduce into said drug a keto group, an aldehyde group, a hydroxyl group, a primary amine group or secondary amine group, a carboxyl group.
